Europäisches Patentamt

European Patent Office

Office européen des brevets

(11) Numéro de publication: **0 425 385 A1**

# (12) DEMANDE DE BREVET EUROPEEN

(21) Numéro de dépôt: 90403016.0

(22) Date de dépôt: 25.10.90

(51) Int. Cl.⁵: **G01N 33/18, G01N 31/22**

(30) Priorité: **27.10.89 FR 8914130**

(43) Date de publication de la demande:
**02.05.91 Bulletin 91/18**

(84) Etats contractants désignés:
**AT BE CH DE DK ES FR GB GR IT LI LU NL SE**

(71) Demandeur: **RHONE-POULENC SANTE**
**20, avenue Raymond Aron**
**F-92160 Antony(FR)**

(72) Inventeur: **Gernez, Gérard**
**59 rue de la Garenne**
**F-91360 Villemoisson Sur Orge(FR)**

(74) Mandataire: **Pilard, Jacques et al**
**RHONE-POULENC SANTE, Service Brevets,**
**20 Avenue Raymond Aron**
**F-92165 Antony Cédex(FR)**

(54) **Procédé pour la détermination de la teneur en iode d'une eau potable.**

(57) Procédé pour déterminer la teneur en iode d'une eau potable par comparaison de la coloration de l'eau à doser à celle d'une solution témoin d'iode en présence d'une quantité déterminée de cérium et d'arsenic et dispositifs pour sa mise en oeuvre.

FIGURE 1

EP 0 425 385 A1

# PROCEDE POUR LA DETERMINATION DE LA TENEUR EN IODE D'UNE EAU POTABLE

La présente invention concerne un procédé pour déterminer la teneur en iode d'une eau potable.

Chez l'homme, la carence ou la déficience en iode conduit à des troubles pathologiques particulièrement importants dont les principaux sont, d'une part, le goître et ses complications (troubles de la déglutition, troubles respiratoires, cancérisation, circulation collatérale) et, d'autre part, l'hypothyroïdie et ses complications (crétinisme, désordres cérébraux, accouchements prématurés, fausses couches, anomalies congénitales).

Si la carence en iode a disparu des pays industrialisés, il n'en est pas de même dans les pays en développement (pays d'Amérique latine le long de la Cordillère des Andes, pays non côtiers d'Afrique et d'Asie).

Parmi les différentes méthodes proposées pour remédier à cette déficience, la plus efficace consiste à ajouter de l'iode à l'eau à usage domestique (boisson, lavage, irrigation) qui le plus souvent est apportée par les puits et les forages.

De nombreuses études ont montré qu'un apport journalier d'environ 100 $\mu$g d'équivalent iode par jour et par personne serait suffisant pour prévenir le développement d'un goître endémique.

Sachant qu'un individu absorbe en moyenne 2 litres d'eau par jour, il est souhaitable qu'un litre d'eau traitée contienne au moins 50 $\mu$g par litre d'iode.

Il existe divers systèmes permettant la libération contrôlée d'iode dans l'eau des puits ou des forages et il est particulièrement important de pouvoir contrôler la teneur effective en iode dans l'eau.

Selon la présente invention, il a été trouvé un procédé oui permet à des personnes, n' ayant aucune connaissance scientifique particulière, d'évaluer sur le terrain, en quelques minutes, avec un minimum de manipulations et en toute sécurité, si la teneur en iode d'une eau potable est supérieure ou égale à 50 par litre.

Selon l'invention le procédé consiste à comparer la coloration de l'eau à doser à celle d'une solution témoin d'iode en présence d'une quantité déterminée de cérium et d'arsenic.

Le procédé selon l'invention est osé sur une méthode colorimétrique découlant de la mise en oeuvre de la réaction de Sandell Kolthoff dont les étapes sont les suivantes:

$$2 Ce^{4+} + 2I^- \rightarrow 2 Ce^{3+} + I_2$$
$$2 Ce^{4+} + I_2 \rightarrow 2 Ce^{3+} + 2I^+$$
$$2 I^+ + As^{3+} \rightarrow I_2 + As^{5+}$$

Sachant que l'ion $Ce^{4+}$ est jaune en solution et que l'ion $Ce^{3+}$ est incolore, la présence d'iode dans le milieu conduira à une diminution de la coloration de la solution initiale contenant les ions $Ce^{4+}$. Ainsi la comparaison de la coloration de l'eau à doser en présence de cérium et d'arsenic à celle de témoins, au bout d'un temps déterminé, permettra de déterminer la teneur en iode de l'eau analysée.

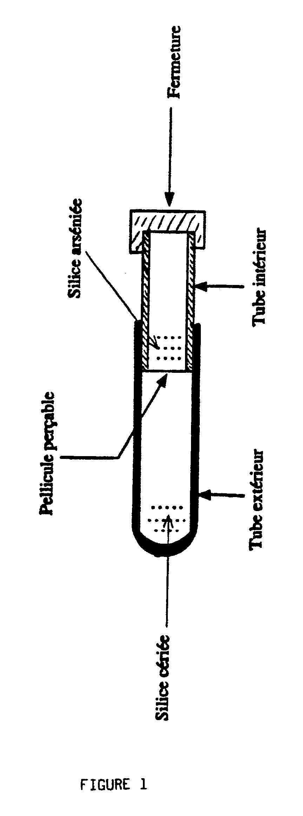

La mise en oeuvre du procédé selon l'invention peut être réalisée au moyen d'un dispositif constitué de deux tubes transparents incolores coaxiaux:
- un tube extérieur, fermé à une extrémité, contient un support (silice) contenant une quantité déterminée de dérivé du cérium (sulfate de cérium et d'ammonium hydraté),
- un tube intérieur, dont le diamètre extérieur correspond au diamètre intérieur du tube extérieur, qui est enfoncé partiellement dans le tube extérieur. Le tube intérieur est fermé à son extrémité située à l'intérieur du tube extérieur par une pellicule perçable et il contient un support (silice) contenant une quantité déterminée de dérivé de l'arsenic (anhydride arsénieux). La partie extérieure du tube intérieur est fermée par un système de fermeture amovible. Le dispositif est représenté par la figure 1.

Généralement, le tube extérieur a un diamètre voisin de 1 cm, l'épaisseur de la paroi étant voisine de 1 mm. La longueur du tube extérieur entre son extrémité fermée et la pellicule perçable est voisine de 6 cm.

Les tubes extérieur et intérieur peuvent être en verre ou en matière plastique transparente rigide et incolore (polystyrène). Il est indispensable que le matériau constituant les tubes soit insensible aux réactifs et à l'iode.

Le dispositif décrit ci-dessus fonctionne de la manière suivante:

On retire la fermeture amovible du tube intérieur puis introduit une quantité déterminée (1 cm3) d'eau à tester à l'aide d'un tube rigide et pointu, perce la pellicule, remet la fermeture amovible et agite soigneusement. Après un repos de quelques minutes, on compare la coloration de la solution obtenue à celle de témoins.

Le support contenant le dérivé du cérium peut être préparé de la manière suivante :

a) Dans une fiole jaugée de 100 cm3, on introduit 2,50 g de sulfate de cérium et d'ammonium dihydraté pur pour analyses et 56 cm3 d'acide sulfurique 0,5M. On complète à 100 cm3 par de l'eau bipermutée. b) Dans un ballon d'évaporateur rotatif on introduit 5 g de silice (TIXOSIL 38 A), 25 cm3 de la solution préparée ci-dessus et 51 cm3 d'acide sulfurioue 0,5M. On évapore à sec à 60°C sous pression réduite (17 mm de mercure ; 2,26 kPa). La masse de résidu doit être légèrement inférieure à 13 g. On complète à 13 g par addition d'eau bipermutée avant d'homogénéiser la poudre obtenue qui est placée au fond du tube extérieur.

Le support contenant le dérivé de l'arsenic peut être préparé de la manière suivante :

a) Dans une fiole jaugée de 100 cm3 on introduit 0,5 g d'anhydride arsénieux pur pour analyses et 0,8 g de chlorure de sodium pur pour analyses. On ajoute 10 cm3 de soude 1M et complète à 100 cm3.

b) Dans un ballon d'évaporateur rotatif, on introduit 5 g de silice (TIXOSIL 38 A) et 25 cm3 de la solution préparée ci-dessus. On évapore à sec à 60°C sous pression réduite (17 mm de mercure ; 2,26 kPa). La masse du résidu obtenu est voisine de 12 g. La poudre obtenue est homogénéisée et placée dans le tube intérieur.

Généralement, lorsque l'on désire déterminer des concentrations en iode voisines de 50 μg par litre, le tube extérieur contient 100 mg de poudre contenant le dérivé du sérium et le tube intérieur contient 100 mg de poudre contenant le dérivé de l'arsenic.

La mise en oeuvre du procédé selon l'invention peut aussi être réalisée au moyen d'un système constitué de 4 tubes en verre ou en matière plastique transparente, rigide et incolore (polystyrène) repérés chacun par un anneau et un bouchon de couleur et d'un tube de prélèvement de 2 cm3.

Les tubes contiennent respectivement les réactifs suivants :
- premier tube : 0,1 cm3 de réactif à l'arsenic dont la préparation est décrite ci-dessous
- deuxième tube : 2 cm3 d'une solution témoin d'iode dont la préparation est décrite ci-dessous
- troisième et quatrième tubes : 200 mg de support silicié contenant le cérium dont la préparation a été décrite précédemment.

Le système fonctionne de la manière suivante :

On prélève 2 cm3 de l'eau à tester à l'aide d'un tube de prélèvement que l'on place dans le tube contenant le réactif à l'arsenic. Le tube est fermé et agité soigneusement par retournements successifs.

Le contenu des premier et deuxième tubes est alors transvasé respectivement dans les troisième et quatrième tubes. Les tubes sont alors bouchés et agités soigneusement par retournements successifs : les suspensions obtenues sont jaunes. On laisse reposer puis on observe la décoloration des suspensions contenues dans les troisième et quatrième tubes. Si la suspension du troisième tube (essai) se décolore plus rapidement que celle du quatrième tube (témoin), l'eau à analyser contient plus de 50 μg/litre d'iode.

Le réactif à l'arsenic peut être préparé de la manière suivante :

Dans une fiole jaugée de 100 cm3, on introduit 5,0 g d'anhydride arsénieux pur pour analyses et 8,09 g de chlorure de sodium pur pour analyses. On ajoute 100 cm3 de soude 10M et complète à 100 cm3 avec de l'eau bipermutée.

La solution témoin d'iode peut être préparée de la manière suivante :

Dans une fiole jaugée de 100 cm3, on introduit 5 cm3 du réactif à l'arsenic et 5 cm3 d'une solution d'iode de référence à 1 mg/litre. On complète à 100 cm3 avec de l'eau permutée.

La présente invention concerne également un nécessaire pour la détermination de la teneur en iode d'une eau potable.

Le nécessaire de dosage peut être constitué soit :
a) - de deux tubes transparents incolores coaxiaux décrits précédemment
- d'un tube rigide et pointu, gradué pour recevoir une quantité déterminée d'eau à analyser ou de solution témoin d'iode
- d'une ampoule ou d'un tube fermé contenant une solution aqueuse stabilisée d'iode à une concentration connue.

Un tube sert à l'analyse de l'eau à doser, le second sert à la réalisation d'un essai témoin avec la solution d'iode de titre connu, soit :
b) de 4 tubes contenant les réactifs décrits ci-dessus et d'un tube de prélèvement.

La comparaison des colorations obtenues avec l'eau à doser et la solution témoin permet de déterminer si la concentration d'iode dans l'eau à doser est inférieure, égale ou supérieure à la concentration de la solution témoin.

**Revendications**

1 - Procédé pour déterminer la teneur en iode d'une eau potable caractérisé en ce que l'on compare la coloration de l'eau à doser à celle d'une solution témoin d'iode en présence d'une quantité déterminée de cérium et d'arsenic.

2 - Dispositif pour déterminer la teneur en iode d'une eau potable selon le procédé de la revendication 1 caractérisé en ce qu'il est constitué de deux tubes transparents coaxiaux,
-un tube extérieur, qui est fermé à une extrémité et qui contient un support contenant une quantité déterminée d'un dérivé du cérium, -un tube intérieur, dont le diamètre extérieur correspond au diamètre intérieur du tube extérieur, qui est enfoncé partiellement dans le tube extérieur, qui est fermé à son extrémité située à l'intérieur du tube extérieur par une pellicule perçable, qui contient un support contenant une quantité déterminée d'un dérivé de l'arsenic et qui est fermé à sa partie extérieure par une fermeture amovible.

3 - Dispositif selon la revendication 2 caractérisé en ce que le tube extérieur à un diamètre voisin de 1 cm et l'épaisseur de la paroi est voisine de 1 mm.

4 - Dispositif selon la revendication 2 caractérisé en ce que les tubes extérieur et intérieur sont en verre ou en matière plastique transparente, rigide et incolore.

5 - Dispositif selon la revendication 2 caractérisé en ce que le support contenant le dérivé du cérium est constitué de silice imprégnée uniformément par une solution acidifiée de sulfate de cérium et d'ammonium dihydraté pur.

6 - Dispositif selon la revendication 2 caractérisé en ce que le support contenant le dérivé de l'arsenic est constitué de silice imprégnée d'une solution d'anhydride arsénieux.

7 - Nécessaire de dosage pour la détermination de la teneur en iode d'une eau potable caractérisé en ce qu'il est constitué:

-de deux dispositifs selon l'une des revendications 2 à 6

- d'un tube rigide et pointu gradué

- d'une ampoule ou d'un tube contenant une solution aqueuse stabilisée d'iode à une concentration connue.

8 - Dispositif pour déterminer la teneur en iode d'une eau potable caractérisé en ce qu'il est constitué de 4 tubes repérés chacun par un anneau et un bouchon de couleur, le premier tube contenant une quantité déterminée de réactif à l'arsenic, le second tube contenant une quantité déterminée d'une solution témoin d' iode et les troisième et quatrième contenant une quantité déterminée de réactif au cérium.

9 - Dispositif selon la revendication 8 caractérisé en ce que les tubes ont un diamètre voisin de 1 cm et l'épaisseur de la paroi est voisine de 1 mm.

10 - Dispositif selon la revendication 8 caractérisé en ce que les tubes sont en verre ou en matière plastioue transparente, rigide et incolore.

11 - Dispositif selon la revendication 8 caractérisé en ce que le réactif à l'arsenic est constitué d'une solution basique d'anhydride arsénieux.

12 - Dispositif selon la revendication 8 caractérisé en ce le réactif au cérium est constitué de silice imprégnée uniformément par une solution acidifiée de sulfate de cérium et d'ammonium dihydrate pur.

13 - Dispositif selon la revendication 8 caractérisé en ce que la solution témoin d'iode est constitué de réactif à l'arsenic et d'une solution d'iode de référence.

14 - Nécessaire de dosage pour la détermination de la teneur en iode d'une eau potable caractérisé en ce qu'il est constitué :

- de quatre tubes selon le dispositif de la revendication 8

- d'un tube de prélèvement.

FIGURE 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | "1987 Annual Book of ASTM Standards", vol. 1101, D1246-82a, 1987, pages 522-530, ASTM, Philadelphia, PA, US; "Standard test methods for iodide and bromide in water"<br>* Page 522, colonne 2, paragraphe 2; page 523, colonne 1, paragraphes 2,4; page 524, colonne 1: "13. Procedure" * | 1 | G 01 N 33/18 G 01 N 31/22 |
| A | IDEM | 2,7 | |
| X | CHEMICAL GEOLOGY, vol. 23, 1978, pages 255-265; R. FUGE et al.: "An automated method for the determination of iodine in geochemical samples"<br>* Figures 1,2; page 256, paragraphe 3; page 258, paragraphe 4 - page 259, paragraphe 1 * | 1 | |
| A | ANALYST, vol. 107, no. 10, décembre 1982, pages 1117-1121; S.D. JONES et al.: "Determination of total iodine and iodate-iodine in natural freshwater"<br>* Figure 2; page 1418; page 1421, paragraphe 2 * | 1,7 | |
| A | US-A-4 125 376 (RAZULIS)<br>* Figure 1; colonne 1, lignes 10-19; colonne 2, lignes 7-22 * | 2 | DOMAINES TECHNIQUES RECHERCHES (Int. Cl.5)<br><br>G 01 N |
| A | DE-A-3 246 132 (VLCEK)<br>* Figure 1; page 2, paragraphe 3; page 3, lignes 13-23; page 4, lignes 9,10; page 8, paragraphe 1; page 12, paragraphe 2; page 10, lignes 14-19 * | 2 | |
| A | US-A-3 705 012 (MARMOR et al.)<br>* Figure 2; colonne 1, lignes 21-29; colonne 3, lignes 3-13 * | 2 | |
| A | US-A-3 932 222 (DORN)<br>* Figures 1,4; colonne 3, lignes 13-39 * | 2,7 | |

Le présent rapport de recherche a été établi pour toutes les revendications

| Lieu de la recherche | Date d'achèvement de la recherche | Examinateur |
|---|---|---|
| La Haye | 01 février 91 | ZINNGREBE U. |